# EUROPÄISCHE PATENTSCHRIFT

(11) **EP 1 237 892 B1**
(45) Veröffentlichungstag und Bekanntmachung des Hinweises auf die Patenterteilung: **23.04.2003**
(21) Anmeldenummer: 00985110.6
(22) Anmeldetag: 29.11.2000
(51) Int. Cl.: C07D 487/04, A61K 31/55, A61P 37/08, A61P 11/06

(54) **VERFAHREN ZUR HERSTELLUNG VON EPINASTINE-HYDROCHLORID IN DER HOCHSCHMELZENDEN KRISTALLMODIFIKATION**
METHOD FOR PRODUCING EPINASTINE HYDROCHLORIDE IN THE HIGH-MELTING CRYSTAL MODIFICATION
PROCEDE DE PREPARATION D'HYDROCHLORURE D'EPINASTINE DANS LE CADRE DE LA MODIFICATION CRISTALLINE A POINT DE FUSION ELEVE

(30) Priorität: 03.12.1999 DE 19958460
(43) Veröffentlichungstag der Anmeldung: 11.09.2002
(73) Patentinhaber: Boehringer Ingelheim Pharma GmbH & Co.KG, 55218 Ingelheim am Rhein (DE)
(72) Erfinder: DACH, Rolf, 55435 Gau-Algesheim (DE)
(86) Internationale Anmeldenummer: EP0011942
(87) Internationale Veröffentlichungsnummer: WO01040229

(56) Entgegenhaltungen:
- EP-A- 0 035 749
- EP-A- 0 496 306

## Beschreibung

Die Erfindung betrifft ein Verfahren zur Herstellung von Epinastine-Hydrochlorid in der hochschmelzenden Kristallmodifikation.

### Hintergrund der Erfindung

Die Verbindung Epinastine (3-Amino-9,13b-dihydro-1H-dibenz-[c,f]imidazol[1,5-a]azepin) gehört zur Gruppe der 2-Aminoimidazoline und stellt eine therapeutisch wirksame Substanz dar, die sich vor allem durch ihre antiallergische und antihistaminerge Wirkung auszeichnet (EP 35749).

Verfahren zur Herstellung von Epinastine-Hydrochlorid sind aus dem Stand der Technik bekannt. In der EP 35749 wird Epinastine-Hydrochlorid durch Fällung aus einer methanolischen Lösung mit Ether erhalten. Die DE 41 02 148 offenbart die Bildung von Epinastine-Hydrochlorid durch Umsetzung der freien Epinastine-Base mit HCI in Dimethylformamid. Vorstehend genannte, aus dem Stand der Technik bekannte Verfahren zur Herstellung des Epinastine-Hydrochlorids weisen allerdings Nachteile auf. So ist durch die genannten Verfahren Epinastine-Hydrochlorid nicht immer in Reinform darstellbar oder fällt in verschiedenen Kristallmodifikationen an. Diesbezüglich bekannt ist eine Modifikation, die bei ca. 250° bis 263°C schmilzt (niedrigschmelzende Kristallmodifikation) und eine andere, die bei ca. 275-281°C schmilzt (hochschmelzende Kristallmodifikation). Durch die in der EP 35749 vorgeschlagene Verwendung von Alkoholen bei der Ausfällung von Epinastine-Hydrochlorid kommt es zu einem Qualitätsverlust durch allmähliche Zersetzung des Produkts. Das Produkt enthält bei diesem Verfahren auch laut DSC (DSC = Differential Scanning Calorimetry) bis zu etwa 5 % der niedrigschmelzenden Kristallmodifikation. Das durch DE 41 02 148 offenbarte Verfahren zur Herstellung des Epinastine-Hydrochlorids beinhaltet die Verwendung von Dimethylformamid, was aufgrund seines hohen Siedepunkts bei der Trocknung nur bei hohen Temperaturen aus dem Verfahrensprodukt entfernt werden kann. Dies führt dazu, daß das Produkt teilweise zu schmelzen beginnt und sich verfärbt. Auch bedarf es für die Trocknung in großtechnischem Maßstab eines unerwünscht hohen Energieaufwands. Schließlich wird Dimethylformamid als fruchtschädigend eingestuft, so daß seine Präsenz in einem Arzneimittel unter allen Umständen zu vermeiden ist.

Es ist Aufgabe der vorliegenden Erfindung, ein Verfahren zur Herstellung von Epinastine-Hydrochlorid bereitzustellen, bei dem die bei den aus dem Stand der Technik bekannten Verfahren auftretenden Nachteile vermieden werden.

### Detaillierte Beschreibung der Erfindung

Die Erfindung betrifft ein Verfahren zur Herstellung von Epinastine-Hydrochlorid (Formel I) (3-Amino-9,13b-dihydro-1H-dibenzo-[c,f]imidazol[1,5-a]azepinhydrochlorid) in der hochschmelzenden Kristallmodifikation ausgehend von Epinastine-Base (II) gemäß (Schema 1).

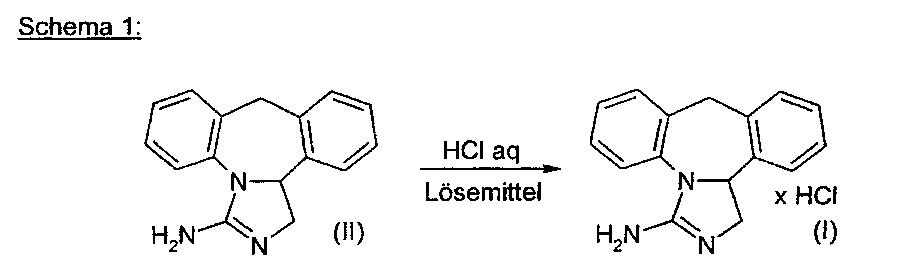

Es ist eine weitere Aufgabe der vorliegenden Erfindung, ein Verfahren zur Herstellung von Epinastine-Hydrochlorid bereitzustellen, welches zur alleinigen Bildung von Epinastine-Hydrochlorid in der hochschmelzenden Kristallmodifikation führt. im Hinblick auf die pharmazeutische Verwendung von Epinastine-Hydrochlorid in Verbindung mit den gesetzlichen Vorschriften zur Qualitätskontrolle pharmazeutischer Produkte ist es zwingend erforderlich, daß Epinastine-Hydrochlorid enthaltende Arzneimittel diesen Wirkstoff nur in einer einzigen Kristallmodifikation enthalten.

Das erfindungsgemäße Verfahren betrifft die Herstellung von Epinastine-Hydrochlorid der Formel (I) 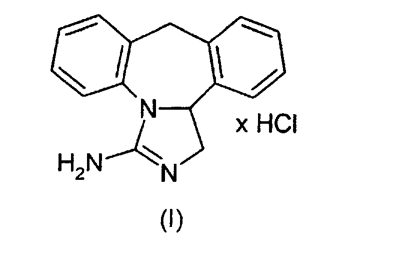 durch ein Verfahren gekennzeichnet, umfassend die folgenden Schritte:
- einen ersten Schritt, bei dem Epinastine-Base der Formel (II) 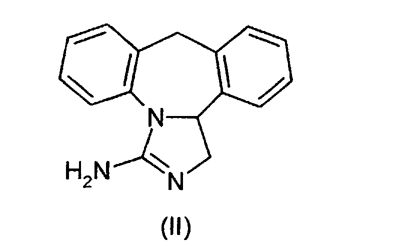 in Wasser suspendiert wird und durch Zugabe von wässriger Salzsäure bei einem pH-Wert ≥7 gelöst wird,
- einen zweiten Schritt, bei dem die aus dem ersten Schritt zugängliche wäßrige Lösung mit einem organischen, nicht mit Wasser mischbaren Lösungsmittel extrahiert wird und das Extraktionsmittel anschließend entfernt wird und
- einen dritten Schritt, bei dem das Produkt der Formel (I) aus der durch den zweiten Schritt zugänglichen wässrigen Lösung durch Zugabe von Salzsäure bei einem pH-Wert ≤6 gefällt und getrocknet wird.

Erfindungsgemäß wird in bevorzugter Art und Weise wie folgt vorgegangen. In einem geeignet dimensionierten Reaktionskessel wird Epinastine Base (II) in Wasser suspendiert und zwischen 20 und 90°C, bevorzugt 40 bis 80°C, besonders bevorzugt bei 50 bis 70°C durch Zugabe von wässriger Salzsäure bei einem pH-Wert, der 7 nicht unterschreiten sollte, gelöst. Dabei ist darauf zu achten, den pH-Wert sauer genug einzustellen, daß sich die Epinastine Base (II) im Wasser löst, aber der pH-Wert dennoch basisch genug bleibt, daß das Epinastine-Hydrochlorid (I) noch nicht ausfällt. Bevorzugt liegt der pH-Wert der Lösung zwischen 7,5 und 9, besonders bevorzugt ist ein pH-Wert von 8. Die Salzsäure wird bevorzugt in konzentrierter Form eingesetzt, wobei unter dem Begrirff "konzentrierte Form" eine ca. 32 Gew.%ige wäßrige Salzsäure verstanden wird.

Die so erhaltene Lösung wird anschließend mit dem organischen, nicht mit Wasser mischbaren, Lösungsmittel extrahiert. Als Lösungsmittel eignen sich beispielsweise halogenierte Kohlenwasserstoffe, wie Methylenchlorid, Tetrachlorkohlenstoff, Ester wie beispielsweise Essigsäureethylester oder Essigsäurebutylester, Ether, wie Dimethyl- oder Diethylester oder andere aus dem Stand der Technik zum Extrahieren von wässrigen Phasen bekannte organische Lösungsmittel. Bevorzugt werden organische Ester eingesetzt, ganz besonders bevorzugt Butylacetat.

Die Lösung wird mindestens einmal, bevorzugt mehrmals mit dem organischen Lösungsmittel extrahiert. Nach Abtrennen des organischen Lösungsmittels werden verbleibende Reste des organischen Lösungsmittels in der wässrigen Phase durch Destillation azeotrop entfernt. Daher werden als organische Lösungsmittel zum Extrahieren bevorzugt solche verwendet, die sich aus Wasser mittels azeotroper Destillation entfemen lassen.

Der so erhaltenen wässrigen Lösung wird im Warmen Aktivkohle zugesetzt, die nach einiger Zeit abfiltriert wird. Bevorzugt wird das Gemisch bei einer Temperatur von ≥50°C und 100°C einige Zeit gerührt, bevorzugt zwischen 70°C und 95°C, ganz besonders bevorzugt bei 80°C bis 90°C.

Nach Filtration wird die klare Lösung auf unter 50°C abgekühlt und mit Salzsäure, bevorzugt konzentrierter Salzsäure ein pH-Wert von ≤6 eingestellt. Bevorzugt wird ein pH-Wert von 3 bis 5 eingestellt, besonders bevorzugt von 3,5 bis 4,5. Die Temperatur der Lösung sollte dabei 25°C nicht unterschreiten, um ein vorschnelles Auskristallisieren des Produkts zu vermeiden. Bevorzugt wird die Temperatur zwischen 30°C und 40°C gehalten. Sobald der gewünschte pH-Wert eingestellt ist, läßt man die Lösung vorsichtig unter Rühren abkühlen, bevorzugt auf ca. 20°C. Nach einigen Minuten fällt das Produkt (I) unter Wärmeentwicklung, die durch Kühlen abgefangen werden sollte, spontan aus.

Nach abgeschlossener Kristallisation, die gegebenenfalls durch Abkühlen beschleunigt und/oder vervollständigt werden kann, wird der Kristallbrei abfiltriert und mit Wasser gewaschen. Bevorzugt wird Eiswasser zum Waschen eingesetzt. Anschließend wird das Produkt getrocknet.

Eventuell noch vorhandenes, nicht ausgefallenes Produkt im Filtrat kann nach den aus dem Stand der Technik bekannten Methoden nachgefällt werden.

Bei dem durch das Verfahren erhaltenen Produkt handelt es sich gemäß den gängigen Analysenmethoden um reines Epinastine-Hydrochlorid (I) in der höherschmelzenden Kristallmodifikation.

Durch das erfindungsgemäße Verfahren sind Ausbeuten des Produkts (I) in der höherschmelzenden Kristallmodifikation von über 80% der Theorie zugänglich.

Entspricht die Reinheit des Produkts nicht den gewünschten Erfordernissen, kann das Verfahren wiederholt werden. Gegebenenfalls werden die letzten Teilschritte wiederholt.

Der Vorteil des erfindungsgemäßen Verfahrens besteht darin, daß gegenüber dem aus dem Stand der Technik bekannten Verfahren, Dimethylformamid vollständig gegen Wasser ersetzt wird. Dadurch wird die pharmazeutische Qualität des Produkts (I) vorteilhaft verbessert, weil u.a. das Produkt keine Restspuren Dimethylformamid oder seiner Zersetzungsprodukte enthält und das Produkt bei schonenden Temperaturen getrocknet werden kann.

### Beispiel

Epinastine-Base (124,5 g) wird in einer definierten Menge Wasser (390 ml) suspendiert und bei einer Temperatur von 60°C durch Zugabe von 32%iger Salzsäure (ca. 50 ml) auf pH 8 eingestellt. Nach zweimaliger Extraktion der wässigen Lösung mit Butylacetat bei 60°C (150 ml und 75 ml) und Abtrennung der organischen Phase, wird eine Teilmenge Wasser unter Normaldruck abdestilliert, um das restliche Butylacetat azeotrop zu entfernen. Anschließend setzt man dem Rückstand bei ca. 90°C Aktivkohle (LX - Ultra, wasserfeucht) zu, rührt 30 Minuten und filtriert sodann die Produktlösung blank und wäscht die abfiltrierte Kohle mit ca. 12,5 ml Wasser nach. In die blankfiltrierte Lösung wird bei 30 - 40°C Salzsäure bis pH 3,5 - 4,5 eindosiert (ca. 1 ml), auf 20°C abgekühlt und mit Epinastine-Hydrochlorid (kristallwasserhaltig und wasserfeucht) angeimpft. Nach ca. 5 - 10 Min. ist, unter leichtem Temperaturanstieg, ein dicker Kristallbrei entstanden.

Zur vollständigen Kristallisation rührt man noch ca. 30-45 Min. bei 20°C, kühlt danach in 1-2 Stunden auf 0-5°C, rührt noch 30 Min. bei 0-5°C, saugt die Kristalle ab und wäscht mit eiskaltem Wasser (ca. 100 ml) nach.

Die Ausbeute beträgt 86,8% % der Theorie. Durch Gewinnung von Nachausbeute kann sie auf 90 % der Theorie erhöht werden.

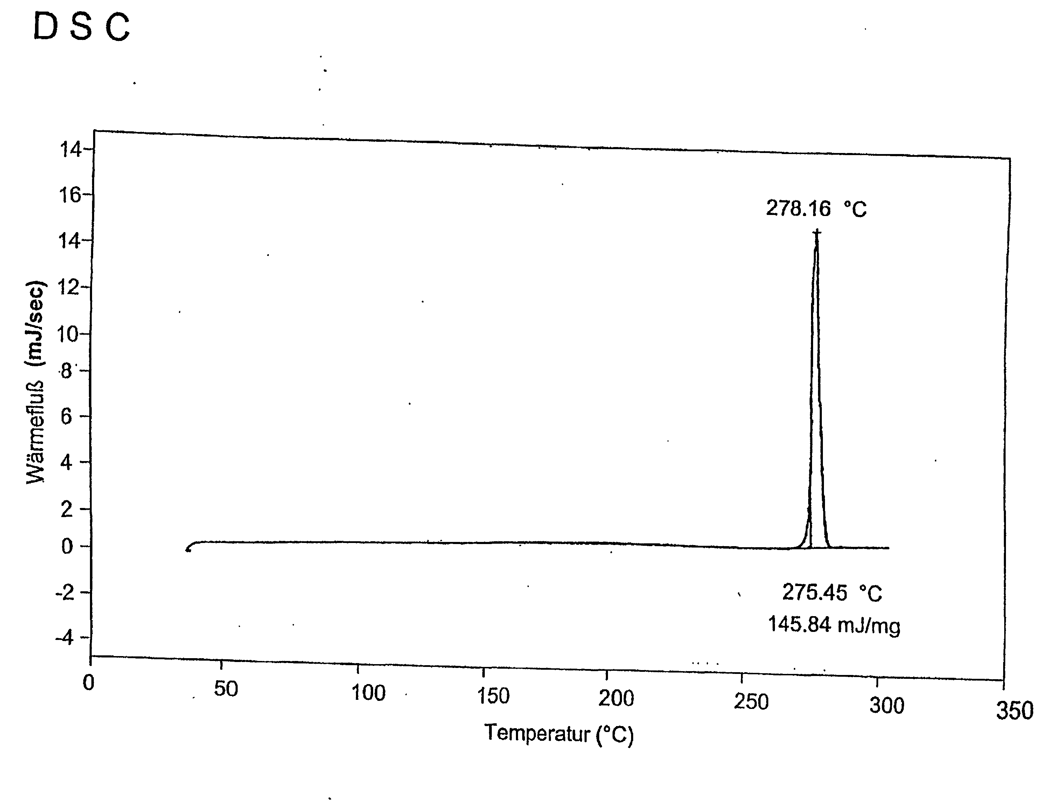

Gemäß dem DSC-Plot nach Figur 1 handelt es sich bei dem erhaltenen Produkt um die gewünschte, hoschschmelzende Modifikation von Epinastine-Hydrochlorid.

## Patentansprüche

1. Verfahren zur Herstellung von Epinastine-Hydrochlorid der Formel (I) 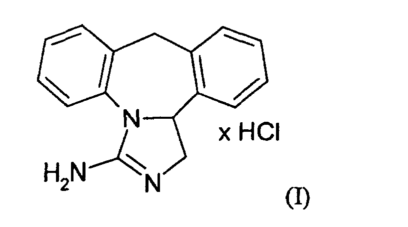 durch ein Verfahren **gekennzeichnet**, das die folgenden Schritte umfasst
- einen ersten Schritt, bei dem Epinastine-Base der Formel (II) 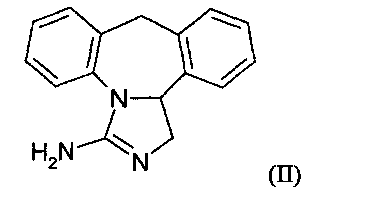
- in Wasser suspendiert wird und durch Zugabe von wässriger Salzsäure bei einem pH-Wert ≥7 gelöst wird,
- einen zweiten Schritt, bei dem die aus dem ersten Schritt zugängliche wäßrige Lösung mit einem organischen, nicht mit Wasser mischbaren Lösungsmittel extrahiert wird und das Extraktionsmittel anschließend entfernt wird und
- einen dritten Schritt, bei dem das Produkt der Formel (I) aus der durch den zweiten Schritt zugänglichen wässrigen Lösung durch Zugabe von Salzsäure bei einem pH-Wert ≤6 gefällt und getrocknet wird.

2. Verfahren gemäß Anspruch 1, **dadurch gekennzeichnet, daß** die Suspension der Epinastine-Base der Formel (II) im ersten Verfahrensschritt bei einer Temperatur zwischen 20 und 90°C, bevorzugt 40 bis 80°C, besonders bevorzugt bei 50 bis 70°C mit der Salzsäure versetzt wird.

3. Verfahren gemäß Anspruch 1 oder 2, **dadurch gekennzeichnet, daß** der pH-Wert während des ersten Verfahrensschritts zwischen 7,5 und 9 liegt, bevorzugt ist ein pH-Wert bei 8.

4. Verfahren gemäß einem der Ansprüche 1, 2 oder 3, **dadurch gekennzeichnet, daß** im zweiten Verfahrensschritt ein halogenierter Kohlenwasserstoff, ein Ether oder ein Ester eingesetzt wird, bevorzugt Essigsäureethylester oder Butylacetat, besonders bevorzugt Butylacetat.

5. Verfahren gemäß einem der Ansprüche 1 bis 4, **dadurch gekennzeichnet, daß** das Extraktionsmittel im zweiten Verfahrensschritt durch Abdekantieren und/oder gegebenenfalls durch azeotrope Destillation entfernt wird.

6. Verfahren gemäß einem der Ansprüche 1 bis 5, **dadurch gekennzeichnet, daß** zwischen dem zweitem und drittem Verfahrensschritt die wäßrige Phase durch Aktivkohle bei einer Temperatur von 50°C und 100°C, bevorzugt von 70°C und 95°C, besonders bevorzugt von 80°C bis 90°C gereinigt wird.

7. Verfahren gemäß einem der Ansprüche 1 bis 6, **dadurch gekennzeichnet, daß** im dritten Verfahrensschritt durch Zugabe der Salzsäure ein pH-Wert von 3 bis 5, bevorzugt von 3,5 bis 4,5 eingestellt wird.

8. Verfahren gemäß einem der Ansprüche 1 bis 6, **dadurch gekennzeichnet, daß** im dritten Verfahrensschritt die Zugabe der Salzsäure bei einer Temperatur von 25°C bis 50°C, bevorzugt 30°C bis 50°C hinzugegeben wird und anschließend die wäßrige Phase abkühlen gelassen wird.

## Claims

1. Process for preparing epinastine hydrochloride of formula (I) 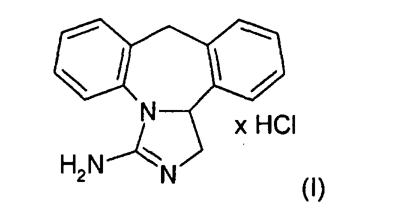 **characterised by** a process which comprises the following steps:
- a first step in which epinastine base of formula (II) 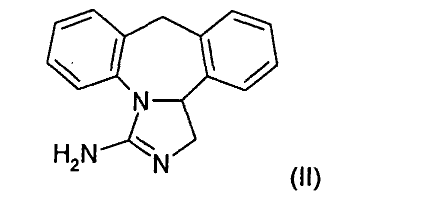 is suspended in water and dissolved by the addition of aqueous hydrochloric acid at a pH of ≥7,
- a second step in which the aqueous solution obtainable from the first step is extracted with an organic, water-immiscible solvent and the extraction agent is subsequently removed and
- a third step in which the product of formula (I) is precipitated from the aqueous solution obtained by means of the second step by the addition of hydrochloric acid at a pH of ≤6 and then dried.

2. Process according to claim 1, **characterised in that** the suspension of the epinastine base of formula (II) is combined with the hydrochloric acid in the first step of the process at a temperature of between 20 and 90°C, preferably 40 to 80°C, most preferably at 50 to 70°C.

3. Process according to claim 1 or 2, **characterised in that** the pH during the first step of the process is between 7.5 and 9, a pH of 8 being preferred.

4. Process according to one of claims 1, 2 or 3, **characterised in that** a halogenated hydrocarbon, an ether or an ester, preferably ethyl acetate or butyl acetate, particularly preferably butyl acetate, is used in the second step of the process.

5. Process according to one of claims 1 to 4, **characterised in that** the extraction agent in the second step of the process is eliminated by decanting and/or optionally by azeotropic distillation.

6. Process according to one of claims 1 to 5, **characterised in that** between the second and third steps of the process the aqueous phase is purified using activated charcoal at a temperature of between 50°C and 100°C, preferably between 70°C and 95°C, particularly preferably from 80°C to 90°C.

7. Process according to one of claims 1 to 6, **characterised in that** in the third step of the process the pH is adjusted to 3 to 5, preferably 3.5 to 4.5, by the addition of hydrochloric acid.

8. Process according to one of claims 1 to 6, **characterised in that** in the third step of the process the hydrochloric acid is added at a temperature of 25°C to 50°C, preferably 30°C to 50°C and then the aqueous phase is left to cool.

## Revendications

1. Procédé de préparation du chlorhydrate d'épinastine de formule (I) 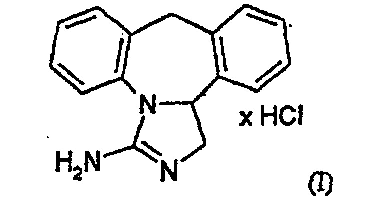 par un procédé **caractérisé en ce qu'**il comprend les étapes suivantes
- une première étape dans laquelle l'épinastine-base de formule (II) 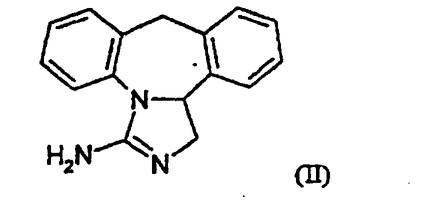
- est mise en suspension dans l'eau et est dissoute par addition d'acide chlorhydrique aqueux à un pH ≥ 7,
- une deuxième étape dans laquelle la solution aqueuse accessible à partir de la première étape est extraite avec un solvant organique non miscible à l'eau et l'agent d'extraction est ensuite retiré et
- une troisième étape dans laquelle le produit de formule (I) est précipité dans la solution aqueuse accessible à partir de la deuxième étape par addition d'acide chlorhydrique à un pH ≤ 6 et est séché.

2. Procédé selon la revendication 1 **caractérisé en ce que** la suspension de l'épinastine-base de formule (II) dans la première étape du procédé est additionnée d'acide chlorhydrique à une température entre 20 et 90°C, de préférence de 40 à 80°C, de manière particulièrement préférée à 50 à 70°C.

3. Procédé selon la revendication 1 ou 2 **caractérisé en ce que** le pH est situé entre 7,5 et 9 pendant la première étape du procédé, un pH de 8 étant préféré.

4. Procédé selon l'une des revendications 1, 2 et 3 **caractérisé en ce que**, dans la deuxième étape du procédé, on utilise un hydrocarbure halogéné, un éther ou un ester, de préférence l'acétate d'éthyle ou l'acétate de butyle, de manière particulièrement préférée l'acétate de butyle.

5. Procédé selon l'une des revendications 1 à 4 **caractérisé en ce que** l'agent d'extraction est retiré dans la deuxième étape du procédé par décantation et/ou éventuellement par distillation azéotropique.

6. Procédé selon l'une des revendications 1 à 5 **caractérisé en ce que**, entre la deuxième et la troisième étape du procédé, la phase aqueuse est purifiée par le charbon actif à une température de 50°C à 100°C, de préférence de 70°C à 95°C, de manière particulièrement préférée de 80°C à 90°C.

7. Procédé selon l'une des revendications 1 à 6 **caractérisé en ce que**, dans la troisième étape du procédé, on établit un pH de 3 à 5, de préférence de 3,5 à 4,5, par addition d'acide chlorhydrique.

8. Procédé selon l'une des revendications 1 à 6 **caractérisé en ce que**, dans la troisième étape du procédé, l'addition d'acide chlorhydrique a lieu à une température de 25°C à 50°C, de préférence de 30°C à 50°C, après quoi la solution aqueuse est mise à refroidir.
